(19) Europäisches Patentamt
European Patent Office
Office européen des brevets

(11) **EP 1 462 101 B1**

(12) **EUROPEAN PATENT SPECIFICATION**

(45) Date of publication and mention of the grant of the patent:
**07.03.2007 Bulletin 2007/10**

(51) Int Cl.:
*A61K 31/192* (2006.01)    *A61K 9/08* (2006.01)
*A61P 29/00* (2006.01)

(21) Application number: **02796784.3**

(22) Date of filing: **11.12.2002**

(86) International application number:
**PCT/ES2002/000590**

(87) International publication number:
**WO 2003/053430 (03.07.2003 Gazette 2003/27)**

(54) **DRINKABLE PREPARATION COMPRISING KETOPROFEN AND USE THEREOF IN THE SIMULTANEOUS TREATMENT OF A GROUP OF ANIMALS OF RESPIRATORY DISEASES**

DRINKBARE ZUBEREITUNG MIT KETOPROFEN UND DEREN VERWENDUNG IN DER SIMULTANBEHANDLUNG EINER GRUPPE VON TIEREN VON ATEMWEGKRANKHEITEN

PREPARATION BUVABLE COMPRENANT DU KETOPROFENE ET UTILISATION DANS LE TRAITEMENT DE MALADIES RESPIRATOIRES DANS UN GROUPE D'ANIMAUX DE MANIERE SIMULTANEE

(84) Designated Contracting States:
**AT BE BG CH CY CZ DE DK EE ES FI FR GB GR IE IT LI LU MC NL PT SE SI SK TR**
Designated Extension States:
**LT LV RO**

(30) Priority: **11.12.2001 ES 200102744**

(43) Date of publication of application:
**29.09.2004 Bulletin 2004/40**

(73) Proprietor: **LABORATORIOS DEL DR. ESTEVE, S.A.**
**08041 Barcelona (ES)**

(72) Inventors:
• **HOMEDES BEGUER, Josep**
**08041 BARCELONA (ES)**
• **SOLANAS IBARRA, Pedro, Juan**
**08014 BARCELONA (ES)**
• **L PEZ CABRERA, Antonio**
**08041 BARCELONA (ES)**
• **LIZCANO GARC A, Javier**
**08041 BARCELONA (ES)**

(74) Representative: **Carpintero Lopez, Francisco et al**
**Herrero & Asociados, S.L.**
**Alcalá 35**
**28014 Madrid (ES)**

(56) References cited:
**EP-A- 1 226 831**       **WO-A-95/07103**
**WO-A-97/45113**       **US-A- 2002 169 212**

## Description

### FIELD OF THE INVENTION

The invention concerns an oral simultaneous coadjuvant treatment to antibacterial treatment for respiratory diseases in a herd of animals using a drink preparation comprising drinking water for animals or artificial milk for suckling animals, supplemented with ketoprofen, for being simultaneously ingested by a herd of animals.

### BACKGROUND OF THE INVENTION

Ketoprofen [2-(3-benzoylphenyl)propionic acid] is an active ingredient that inhibits cyclooxygenase with analgesic, antiinflammatory and antipyretic properties.

Document EP769294 discloses pharmaceutical compositions comprising ketoprofen that are provided as ready-to-drink liquids or in dry form to be dissolved in water before consumption. These compositions are intended to be administered to human patients as pharmaceutical compositions for the treatment of pain and fever.

The use of ketoprofen (racemic) has been also described in veterinary applications and a number of veterinary products contain ketoprofen either as tablets for oral administration for dogs or as an injectable solution for cattle, pigs and horses. Both these preparations are for strictly individual use and animals must be treated one by one both when reared individually and also when reared in herds (farms).

Therefore, document WO95/07103 discloses compositions obtained from ketoprofen solid by incorporating it together with other agents into an aqueous-based orally acceptable carrier, administered to a mammalian organism, for the individual treatment of cold, cold-like and/or flu symptoms.

In modem intensive farming, non-dairy livestock, for example pigs, beef cattle, etc. are reared in large herds that can hold up to thousands of animals. In these farms, the animal population density is very high and when a focus of infection appears this rapidly spreads to the whole herd (or at least the whole stockyard), especially in the case of respiratory infections. In these cases, the whole herd should be treated simultaneously. If these are treated with the ketoprofen products currently available on the market, each individual animal must be held still and administered the appropriate dose of the medicine and the whole process repeated for several days. This is very time-consuming and requires considerable manual work, increasing greatly the cost of the treatment, which must be added to the cost of the antibiotic treatment administered simultaneously, and causing animal stress that greatly worsens the course of the illness, making this type of treatment unviable in practise.

### SUMMARY OF THE INVENTION

The invention concerns a solution for the problem of developing a system to simultaneously treat a herd of animals with ketoprofen.

The solution provided by the invention is based on the inventor's observation that by adding an aqueous solution of ketoprofen to a herd's drinking water or to the artificial milk for suckling animals in an appropriate amount relative to animal's bodyweight and the number of animals present, a herd of animals can be easily treated simultaneously.

This invention is advantageous because individual animals do not have to be held still to administer them the treatment, avoiding delays in administering the product and reducing manual labor costs and stress to animals.

One object of this invention is a drink preparation comprising the animal drinking water or artificial milk for suckling animals supplemented with ketoprofen.

Another additional object of this invention is a method for obtaining said drink preparation comprising ketoprofen.

Another additional object of this invention is the use of an aqueous solution of ketoprofen for the obtention of this drink preparation comprising ketoprofen for the simultaneous coadjuvant treatment to antibacterial treatment of a respiratory disease, in a herd of animals.

### BRIEF DESCRIPTION OF THE FIGURES

Figure 1 shows a diagram illustrating the experimental design of a study of the clinical efficacy of a 3% aqueous solution of ketoprofen to complement the antibiotic treatment of Bovine Respiratory Disease (BRD) in calves (Example 2) and in suckling calves (Example 3).

Figure 2 is a graph that shows the evolution of the clinical index of each group of calves during the treatment.

Figure 3 is a graph, which shows the temperature profile of each group of calves during treatment.

Figure 4 is a graph that shows the change in clinical index of each group of suckling calves during treatment.

Figure 5 is a graph that shows the temperature profile of each group of suckling calves during treatment.
Figure 6 is a graph that shows the rectal temperature profile in each group of pigs over treatment.
Figure 7 is a graph that shows the change in clinical index for each group of pigs over treatment.

**DETAILED DESCRIPTION OF THE INVENTION**

The invention concerns a drink preparation comprising animal drinking water or artificial milk for suckling animals supplemented with ketoprofen, and comprising components as listed in claim 17 or claim 19.

The drink preparation comprising ketoprofen provided by this invention, hereinafter referred as the drink preparation of the invention, is a preparation that can be drunk by animals.

The drinking water for animals and artificial milk for suckling animals are known products. For the practical application of this invention, any animal drinking water or artificial milk for suckling animals can be used.

Ketoprofen is a commercially available product useful as an analgesic, antiinflammatory and/or antipyretic agent. Alternatively, it can be obtained by a method such as that described in the American patent US 3,641,127. In the drink preparation of the invention, ketoprofen is dissolved. Since the drink preparation of the invention is designed to treat a herd of animals, the amount of ketoprofen contained in said drink preparation can vary within a wide interval. In general, the drink preparation of the invention contains ketoprofen in an amount sufficient to provide a therapeutically effective amount to the animal that ingests this drink preparation. As used in this specification, the term "therapeutically effective amount" refers to an amount sufficient to have a therapeutic effect on the animal, for example, an amount ranging from 1 to 5 mg of ketoprofen per kg in weight of the live animal that ingests this drink preparation per day (1-5 mg/kg/day). In one specific application, the drink preparation of the invention contains ketoprofen in an amount sufficient to supply 3 mg/kg/day of ketoprofen to the animal that ingests this drink preparation.

The drink preparation of the invention can be used for the oral treatment of conditions causing fever, inflammation and/or pain, in a herd of animals simultaneously. In the sense used in this specification, the term "conditions that cause fever, inflammation and/or pain" can correspond to any alteration or illness, of infectious origin or not, in which all or some of these symptoms are present. Also, the term "herd of animals" refers to a group of animals, such as, for example the group of animals present in an intensive cattle farm and the term "simultaneously" refers to the fact that the herd of animals receives the ketoprofen treatment at the same time, regardless of whether all the animals ingest the drink preparation of the invention at the same time, since the ketoprofen is incorporated in the drinking water or in the artificial milk for suckling animals.

Examples 2 and 3 demonstrate the clinical efficacy of a drink preparation of the invention as a coadjuvant treatment to the antibiotic treatment of Bovine Respiratory Disease (BRD) in herds of calves or suckling calves. Example 4 illustrates the clinical efficacy of a drink preparation of the invention as a coadjuvant treatment to the antibiotic treatment of Porcine Respiratory Disease Complex (PRDC) in herds of fattening pigs.

The drink preparation of the invention can be obtained by a method that involves mixing an aqueous solution of ketoprofen with animal drinking water or artificial milk for suckling animals. The amount of aqueous solution of ketoprofen mixed with the drinking water or with the artificial milk can vary within a wide interval. The ratio between (i) the aqueous solution of ketoprofen and (ii) the drinking water or artificial milk depends on several factors, including the concentration of ketoprofen in the aqueous solution of ketoprofen, the amount of drinking water or artificial milk drank by the animals and the dose of ketoprofen to be administered to each animal. The amount of water or artificial water drank by the animal is a very variable factor and depends, among other factors, on the animal's weight, the climatic conditions and the animals clinical condition. As an example, a 3% aqueous solution of ketoprofen can be added to the drinking water for animals in an amount ranging from 0.5 to 2 millilitres of this aqueous solution of ketoprofen per litre of drinking water or in an amount of between 1.5 and 7.0 millilitres of this aqueous solution of ketoprofen per litre of artificial milk for suckling animals.

The concentration of ketoprofen in the drinkable preparation of claim 1 can vary within a wide interval. The lower limit is determined by the minimum amount of ketoprofen that it is practical to add to the preparation of aqueous solution of ketoprofen with this intended use. The upper limit is determined, among other factors, by the concentration of ketoprofen in aqueous solution, at a specific pH, at which this begins to precipitate. As an example, the concentration of ketoprofen in aqueous solution can range from 1 % to 15% (w/v), normally between 1% and 5% (w/v). In a particular embodiment, the concentration of ketoprofen in the aqueous solution is 3% (w/v).

Ketoprofen is almost insoluble in water and several methods can be followed to prepare the aqueous solution. One of these consists in using a co-solvent, for example, polyethylene glycol 400, 1-methyl-2-pyrrolidone, etc., and another strategy consists in increasing the solubility of ketoprofen by the formation of salts *in situ* with a basic amino acid, for example lysine, arginine, etc.

In a particular embodiment, the cosolvent used to prepare the aqueous solution of ketoprofen for use in claim 1 is 1-methyl-2-pyrrolidone.

In another particular embodiment, this aqueous solution of ketoprofen includes L-arginine in order to increase

3

the solubility of ketoprofen in water by forming *in situ* the corresponding salt with L-arginine. The concentration of L-arginine is established by adjusting the equimolecular amount of this substance required to solubilise ketoprofen, as well as an additional amount in order to favour the use regimes of the aqueous solution of ketoprofen for different qualities of drinking water or types of artificial milk. The concentration of L-arginine in the aqueous solution can vary within a wide interval depending on the concentration of ketoprofen. As an example, the concentration of L-arginine in the aqueous solution can range from 2% to 30% (w/v), usually between 2% and 8% (w/v). In a particular embodiment, the concentration of L-arginine in the aqueous solution is 5.5% (w/v).

The aqueous solution of ketoprofen for use in claim 1 can also contain one or more veterinary acceptable additives such as humectants, pH modifiers, preservatives, etc.

As a humectant, any compound that promotes the dissolution of ketoprofen in aqueous solution can be used, for example polysorbates, poloxamers, such as poloxamer 407, etc.

It is known (Index Merck, Analytical Profiles of Drug Substances), that the pKa of ketoprofen in acetonitrile/water (3/1) ranges from 5.02 to 5.94 in methanol/water (3/1), being the mean value of about 5.5, while for arginine the $pK_1$ is 2.18 (carboxylic) and the $pK_2$ is 9.09 (amino), being the mean value of about 5.6, and after a pH equal to or higher than 5.5, both compounds are sufficiently ionized (dissociated) to interact and to keep ketoprofen in solution. With this purpose, when making up the aqueous solution, the pH was adjusted from 5.5 to 7.0, by adding a pH modifier agent. Experiments carried out showed that ketoprofen precipitated at a pH lower than 5.5.

Therefore, the pH modifier is a compound, acidic or basic, that provides the aqueous ketoprofen solution with such a pH value that the ketoprofen remains in an ionised form, for example a pH between 5.5 and 7.0, preferably of 6.5. In a particular embodiment, when the aqueous ketoprofen solution contains 1-methyl-2-pyrrolidone as a cosolvent, the pH modifier agent is an inorganic base, for example, sodium hydroxide, in the amount required (q.s.) to provide the aqueous solution with a pH of 6.5. In another particular embodiment, when the aqueous solution of ketoprofen contains a basic amino acid, for example, L-arginine, the pH modifier agent is an acid such as citric, maleic, hydrochloric acid etc. in the required (q.s.) to provide the aqueous solution of ketoprofen with a pH of between 5.5 and 7.0.

The results of the stability studies of the ketoprofen aqueous solution in relation to the solution appearance, as a consequence of the small variation in pH value (a key factor for this use), demonstrate that the goal to maintain ketoprofen in solution is achieved perfectly. The remaining results confirm the success of the formulation.

After diluting the aqueous solution of ketoprofen according to claims 17 or 19 with animal drinking water or artificial milk for suckling animals, in order to obtain the drink preparation of this invention, solubilization of ketoprofen is aided by diluting the product. Therefore, even in relatively acidic waters (around pH 5.0), the drink preparation of the invention has been observed to remain stable and any precipitate forms even after 48 hours in refrigeration. It has also been shown that the pH is not modified when the aqueous solution of ketoprofen is added to the drinking water according to the proposed used and that the chemical data remain unaltered after 72 hours, using the sample directly without any additional treatment, as demonstrated by the results of the stability tests.

Favourably, the aqueous ketoprofen solution includes a preservative in order to ensure its preservation during the proposed use. As a preservative, benzylic alcohol is used, an antimicrobial preservative used in cosmetics, food and a wide range of pharmaceutical formulae including oral and parenteral preparations, at a concentration ranging from 0.5% to 3%, for example between 0.5% and 2.5%. Benzylic alcohol is a product included in the Handbook of Inactive Ingredients of the FDA (injectables, capsules, solutions and oral tablets, and topical and vaginal preparations), and also present in parenteral and non parenteral products sold in Great Britain (Handbook of Pharmaceutical Excipients, 3rd edition, page 42).

The aqueous solution of ketoprofen is obtained by mixing its components in appropriate amounts. This aqueous solution of ketoprofen has the following composition:

| Ingredient | |
|---|---|
| Ketoprofen | 1-15% (w/v) |
| L-Arginine | 2-30% (w/v) |
| Benzylic alcohol | 0.5-3% |
| Anhydrous citric acid (q.s.) | pH between 5.5 and 7.0 |
| Purified water q.s. | 100% |

In another particular embodiment, this aqueous solution of ketoprofen has the following composition:

| Ingredient | |
|---|---|
| Ketoprofen | 1-5% (w/v) |
| L-Arginine | 2-8% (w/v) |

(continued)

| Ingredient | |
| --- | --- |
| Benzylic alcohol | 0.5-2.5% |
| Anhydrous citric acid q.s. | pH between 6.0 and 7.0 |
| Purified water q.s. | 100% |

The following examples serve to illustrate the invention and should by no means be considered to limit its scope of application.

## EXAMPLE 1

### Preparation of an aqueous solution of ketoprofen

Since ketoprofen is almost insoluble in water, several experiments were carried out in an attempt to resolve this problem.

Therefore, in the first place, preliminary experiments were carried out to establish the proportion of cosolvent or basic amino acid required to dissolve 1 g of ketoprofen in an aqueous medium and then the samples were subjected to forced conditions (5 °C and -20 °C). As a co-solvent, polyethylene glycol 400 and 1-methyl-2-pyrrolidone were tested and L-arginine and L-lysine as basic amino acids. From these preliminary tests, 1-methyl-2-pyrrolidone was selected as a co-solvent since ketoprofen is more soluble in this cosolvent than with polyethylene glycol 400, and L-arginine as the amino acid.

Then, further experiments were carried out using the co-solvent and basic amino acid chosen and the possibility of adding a humectant agent (poloxamere 407) was considered to favour ketoprofen solubilization. Therefore, the preformulation step was started by preparing the centesimal compositions recorded in Table 1.

**Table 1**

| Compositions tested | | | | | | |
| --- | --- | --- | --- | --- | --- | --- |
| **Compound** | **1** | **2** | **3** | **4** | **5** | **6** |
| Ketoprofen | 3.000 | 3.000 | 3.000 | 3.000 | 3.000 | 3.000 |
| 1-methyl-2-pyrrolidone | 60.000 | 60.000 | - | - | 30.000 | 30.000 |
| NaOH q.s. | pH 6.5 | - | - | - | pH 6.5 | - |
| Monopotassium phosphate | - | 0.668 | - | - | - | 0.668 |
| Disodium phosphate | - | 0.313 | - | - | - | 0.313 |
| L-Arg | - | - | 7.000 | 7.000 | - | - |
| Citric acid q.s. | - | - | PH 6.5 | pH 6.5 | - | - |
| Poloxamere 407 | - | - | - | 1.000 | 2.000 | 2.000 |
| Purified water q.s. | 100 ml | 100 ml | 100 ml | 100 ml | 100 ml | 100 ml |

The pH was adjusted to 6.5 and it was demonstrated that the compositions based on the formation of salts *in situ,* are pH dependent and for pH values lower than 5.5, obtained by adding diluted hydrochloric acid, precipitation of ketoprofen occurred.

Of the compositions tested, the one identified as composition n. 2 did not have a good solubility and those containing poloxamere 407 (compositions n. 4, 5 and 6) had a tendency to produce foam since this compound is a non ionic surfactant. Due to the high contents (60%) of co-solvent in composition n. 1, composition n. 3 was chosen for the following experiments.

In another group of experiments, it was decided to adjust the amount of L-arginine required to solubilise ketoprofen, to test different preservative agents and, finally, to carry out the appropriate modifications by adding L-arginine, for the solution to be highly compatible with the different water qualities to be treated. The preservative chosen was benzylic alcohol. Taking into account all these considerations the composition described below was obtained, hereinafter referred as "EV solution", that is used in the experiments described in Examples 2,3 and 4.

**Composition of a 3% aqueous solution of ketoprofen (EV solution)**

| Ingredient | |
| --- | --- |
| Ketoprofen | 3.000 g |
| L-Arginine | 5.500 g |
| Benzylic alcohol | 2.000 g |
| Anhydrous citric acid q.s. | pH 6.5 |
| Purified water q.s. | 100 ml |

After, a pilot batch was made with the EV solution and using this batch the stability at 0.1% (v/v) was verified in drinking water (tap water and purified water) for animals and in artificial milk for suckling animals, in order to be able to recommend a maximum using term. The results obtained give a using term of 72 hours for the drinking water containing 0.1 % of the aqueous solution of ketoprofen and 24 hours of using term for the artificial milk for suckling animals containing 0.1% of this aqueous solution of ketoprofen.

## EXAMPLE 2

### STUDY OF THE CLINICAL EFFICACY OF AN AQUEOUS SOLUTION OF KETOPROFEN, ADMINISTERED ORALLY IN DRINKING WATER, AS A COMPLEMENT TO THE ANTIBIOTIC TREATMENT OF RESPIRATORY DISEASE IN CALVES

Most infectious processes are known to cause important inflammation of soft tissues often associated with pain. They are also usually associated with release of endotoxins that cause, among other phenomena, a febrile condition. Together, these phenomena all worsen the animal's clinical condition and prolong the course of the illness [Laval, A. (1992). Utilisation des anti-inflammatoires chez le porc. Rec. Med. Vét. 168(8/9):733-744]. Because of this, elimination of these symptoms by administering non steroid antiinflammatory agents (NSAIDs) as a complement to antibacterial therapy has been described as a very beneficial process in veterinary medicine [Kopcha, M. et al. (1989). Experimental uses of flunixin meglumine and phenylbutazone in food-producing animals. JAVMA 194(1):45-49]

One of the applications of this therapeutic approach is the treatment of Bovine Respiratory Disease (BRD), a pathological complex in whose etiopathogeny different etiological agents, especially *Pasteurella multocida* and *Mycoplasma spp.* [Merck Veterinary Manual (1998), 8<sup>th</sup> ed. Merck & Co., Inc., Whitehouse Station, NJ, USA, p. 2188] have been implicated. More specifically, it has been demonstrated that the use of selected NSAIDs, including ketoprofen, complementary to the antibacterial treatment of this disease increases the recovery rate of the animals and the percentage of clinical improvement at the end of treatment [Deleforge, J. et al. (1994). A field evaluation of the efficacy of tolfenamic acid and oxytetracycline in the treatment of bovine respiratory disease. J. Vet. Pharmacol. Therap. 17:43-47; Balmer, T.V. et al., (1997). Comparison of Carprofen and Flunixin Meglumine as adjunctive therapy in Bovine Respiratory Disease. The Veterinary Journal 154:233-241; Lockwood, P. et al. (1998). Eficacia clinica comparada de tres antiinflamatorios no esteroideos (NSAID), flunixin meglumine, carprofeno y ketoprofeno como complemento del tratamiento antibacteriano de la enfermedad respiratoria bovina. Albeitar 16:15]. However, in most published studies that describe this therapeutic practise, the administration of NSAID has been done parenterally (intramuscular or intravenous), and few studies have used the oral route for this purpose.

In order to assess the clinical efficacy of the EV solution, a 3% aqueous solution of ketoprofen (Example 1) was orally administered in the drinking water of beef calf herds as a coadjuvant treatment to antibacterial treatment of Bovine Respiratory Disease (BRD) and a clinical trial was conducted following the principles of good clinical practise.

For this purpose, this clinical study was designed and carried out as a controlled multicentric clinical trial [the animals included in the study were distributed into two groups, one of which received a treatment based on Antibiotics + EV solution while the other received a treatment based on Antibiotic + Placebo (excipient of EV solution) acting, therefore, as a negative control]. The trial was randomized and blind.

## 1. MATERIAL AND METHODS

### 1.1 ANIMALS

In accordance with the required sample size, a total of 350 young grazing beef cattle in the fattening stage were included (175 in each treatment group). At the time of inclusion, all the calves presented a clinical picture of moderate or severe BRD. During the study, the animals were housed in an out-building and allocated to one of two stockyards (one for each treatment group). All the animals received commercial standard feed without medicinal additives and drinking water *ad libitum* from automatic drinking devices.

**1.2 EXPERIMENTAL DESIGN**

The experimental period lasted a total of 10 days. Selection of animals was done on the first day of the experimental period (day D1). After detecting an outbreak of BRD in a beef calf fattening farm, animals fulfilling the following selection criteria were selected:

1°. With a clinical symptomatology compatible with BRD, with a severity higher than 1.1 (>1.1: moderate or severe disease) on the clinical score scale created *ad hoc* for the study; and
2°. They had not received antibacterial or antiinflammatory agents in the seven days before the selection.

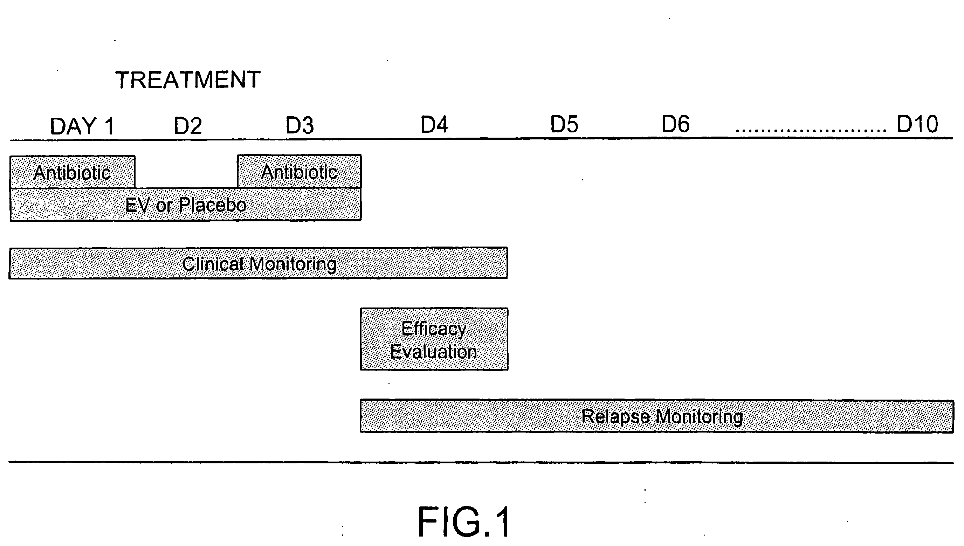

After selecting a batch, the animals were distributed into two homogeneous equally sized groups. Then, each of the groups was placed in a different stockyard and by a simple randomized technique, one group was assigned to treatment A (Antibiotic + EV solution) and the other group to treatment B (Antibiotic + Placebo).
The treatment period lasted a total of 3 days for all animals (days D1 to D3) and treatment consisted in administering two products to each group; an Antibiotic, the same for both groups, on days D1 and D3, and the EV solution (Group A) or its excipient (Group B), on days D1, D2 and D3 (see Figure 1).
Figure 1 shows a diagrammatic representation of the experimental design of the clinical efficacy study of a 3% aqueous solution of ketoprofen used complementarily to antibacterial treatment of BRD in calves.
From the first day (day D1) until the last day of treatment (day D4) the clinical status of the animals was monitored (Figure 1). During this period, the following operations were carried out:

- Measurement of the Clinical Index or severity of the disease: days D1 to D4
- Measurement of the body temperature: days D1 to D4
- Measurement of the body weight: days D1, D4 and D10
- Monitoring for possible relapse: days D4-D10

***Measurement of the Clinical Index or severity of the disease***

This index was calculated individually for each animal, once a day from days D1 to D4, using a score scale based on one used previously by other authors [Raynaud, J.P., et al. (1986). Examen clinique standarisé dans les broncho-pneumonies infectieuses enzootiques des bovins et essais de medicaments-methodologie et interprétation statistique. Proceedings of XIVth World Congress on Cattle Diseases. Dublin 26-29 August pp 435-439; Deleforge (1992) *supra*].
According to the scoring systems described by the previously mentioned authors, a Clinical Index (CI) higher than 1.1 is equivalent to a moderate or severe disease status. On the other hand, a CI equal to or lower than 1.1 is equivalent to no disease or an imperceptible disease status:

$$CI \leq 1.1 \qquad \} \ \text{No or imperceptible disease}$$

$$1.1 < CI < 2 \ \} \ \text{Moderate disease status}$$

$$CI \geq 2 \qquad \} \ \text{Severe disease status}$$

This parameter was used to study the main variable of this study, "CLINICAL EFFICACY" at the end of treatment (day D4), on a dual scale with the categories:

$$\text{POSITIVE EVALUATION = Success of treatment } (CI \leq 1.1),$$

and

## NEGATIVE EVALUATION = Failure of treatment (CI > 1.1).

### Measurement of body temperature

This parameter was measured daily for each animal in the morning on days D1 to D4. This parameter corresponds to one of the secondary variables of the study.

### Measurement of animal weight

The weight of each of the animals was recorded on days D1 (baseline), D4 and D10, using balances specially designed to weigh large animals (EziWeight-1 / TRU-TEST®).

### Monitoring for possible relapse

Finally, over the 7 days after the end of the treatment period (days D4 to D10), the possible appearance of relapses was controlled (Figure 1).

## 1.3 TREATMENTS

### 1.3.1 Characteristics of the products

#### 1.3.1.1 Antibiotics

| NUFLOR® | Pharmaceutical form: | Injectable solution |
|---|---|---|
| | Composition 100 ml: | Florfenicol 30 g |
| | | Excipient q.s. |
| | Manufacturers: | Schering-Plough, S.A. |

#### 1.3.1.2 Products studied

| EV Solution | Pharmaceutical form | Oral solution |
|---|---|---|
| | Composition 100 ml: | Ketoprofen 3 g (Example 1) |
| | | Excipient q.s. |
| | Manufacturers: | Laboratorios Dr. Esteve, S.A. |

| PLACEBO | Pharmaceutical form: | Oral solution |
|---|---|---|
| | Composition 100 ml: | Excipient q.s. (Example 1) |
| Manufacturers: | Laboratorios Dr. Esteve, S.A. | |

### 1.3.2 Dose, therapeutic regime and route of administration

#### 1.3.2.1 Antibiotic

The antibiotic preparation was administered to both groups (A and B) in the dose recommended by the manufacturer by intramuscular injection in the neck muscle. The treatment consisted of 2 doses administered with an interval of 48 hours (days D1 and D3).

### 1.3.2.2 Products studied

Animals from Group A received a mean dose of 1 ml of solution EV / 10 kg of live weight (equivalent to 3 mg ketoprofen / kg / day). Animals from Group B received a mean dose of 1 ml of Placebo / 10 kg / day. Both products were administered orally in the drinking water, daily for three consecutive days (days D1, D2 and D3).

### 1.4 STATISTICAL PROCEDURES

Mean values and profiles of clinical index, temperature and weight in both groups were compared by the Student's t-test and when criteria of normality were not reached the Mann-Whitney Test was applied.

The main study variable, "Treatment efficacy", was studied by comparing the percentages of treatments that had been evaluated positively (success) and those evaluated negatively (failures) in each of the treatment groups, using Pearson's Chi-squared statistical test.

### 2. RESULTS AND DISCUSSION

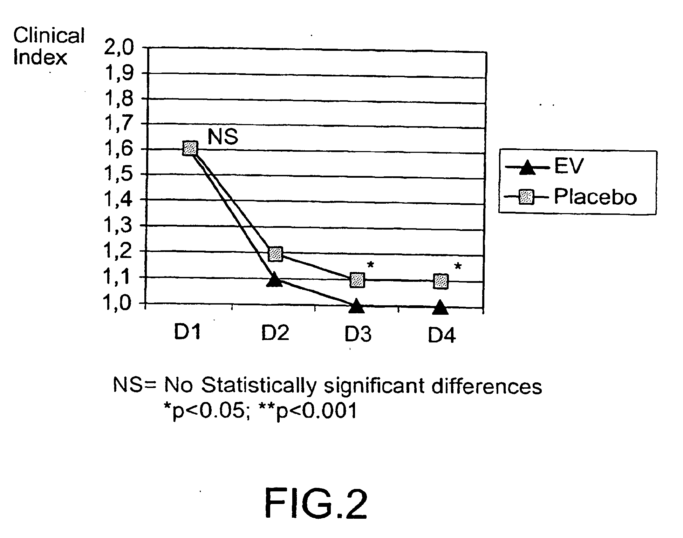

In spite of the fact that the mean basal value of the clinical index was very similar in both groups, during the control carried out on the second day of treatment (D2) this value was significantly lower in Group A (treated with EV solution) than in group B (treated with Placebo), and remained like this until the end of the treatment (Table 2). On the other hand, while Group A by the second day of treatment (day D2) had reached a mean clinical index lower than or equal to 1.1 (equivalent to a low or imperceptible disease status), in Group B this index remained above this value until day D3 (Table 2, Figure 2).

**Table 2**

| Mean values $\pm$ standard deviation of the clinical index of each group, during the treatment | | | |
|---|---|---|---|
| | **GROUP A (EV Solution)** | **GROUP B (Placebo)** | **Statistical comparison** |
| **D1** | 1.6 $\pm$ 0.3 | 1.6 $\pm$ 0.3 | NS (p>0.05) |
| **D2** | 1.1 $\pm$ 0.2 | 1.2 $\pm$ 0.2 | p<0.001 |
| **D3** | 1.0 $\pm$ 0.1 | 1.1 $\pm$ 0.2 | p<0.05 |
| **D4** | 1.0 $\pm$ 0.0 | 1.1 $\pm$ 0.2 | p<0.05 |
| NS = No statistically significant difference between the two groups (p>0.05). | | | |

These results indicate that the symptoms of the disease disappear first in animals from Group A than in animals from Group B, which, according to the mean value of the clinical index on day 4, had not been completely cured by this time (Figure 2).

According to the protocol, using the clinical index presented by each animal in the control group on day D4, the clinical researcher evaluated the efficacy of each treatment (main variable), only considering those in which the clinical index had decreased to a value lower than or equal to 1.1 (low or imperceptible disease status) as being successfully treated. The results of this evaluation are recorded in detail in Table 3.

**Table 3**

| Percentage of animals in which the treatment was evaluated as a success or as a failure in each of the two groups | | | |
|---|---|---|---|
| | **GROUP A (EV Solution)** | **GROUP B (Placebo)** | **Statistical comparison** |
| **SUCCESS Clinical index $\leq$ 1.1** | 172 (98%) | 151 (86%) | p<0.001* |
| **FAILURE Clinical Index > 1.1** | 3(2%) | 24 (14%) | |
| *Chi-squared test = 17.699 with 1 degree of freedom. Statistical power =99.4% | | | |

As reflected in Table 3, the percentage of animals in which the treatment was successful was significantly higher in the group treated with EV solution than in the Group treated with Placebo. The difference between these percentages was 12%, a figure of considerable clinical relevance if we consider that the success percentage predicted for the Placebo group was around 85%, as indeed occurred.

According to the results, administration of the EV solution in the drinking water as a coadjuvant treatment for the antibacterial therapy of BRD has a beneficial therapeutic effect compared to the treatment of this disease with antibiotics alone.

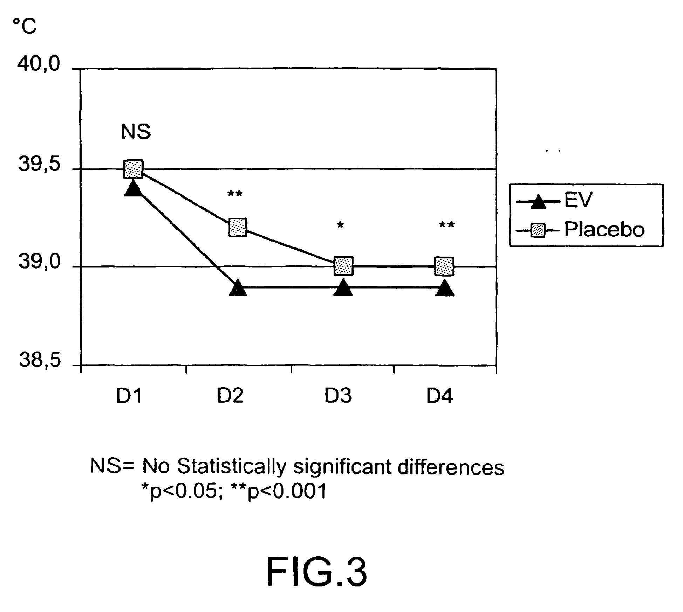

As can be observed in Table 4 and in Figure 3, in spite of a very similar mean basal temperature in both Groups, on the second day of treatment this value was significantly lower in Group A than in Group B, and remained lower until the end of treatment.

**Table 4**

| Mean values $\pm$ standard deviation of the rectal temperature of each group during treatment | | | |
|---|---|---|---|
| | **GROUP A (EV Solution)** | **GROUP B (Placebo)** | **Statistical comparison** |
| **D1** | 39.4 $\pm$ 0.5 °C | 39.5 $\pm$ 0.5 °C | NS |
| **D2** | 38.9 $\pm$ 0.3 °C | 39.2 $\pm$ 0.4 °C | p<0.001 |
| **D3** | 38.9 $\pm$ 0.3 °C | 39.0 $\pm$ 0.3 °C | p<0.05 |
| **D4** | 38.9 $\pm$ 0.3 °C | 39.0 $\pm$ 0.4 °C | p<0.001 |
| NS = No statistical significance between the two groups (p>0.05). | | | |

As can be observed in Figure 3, the drop in temperature in Group A was considerable and mainly occurred on the first day of treatment. In contrast, in Group B this temperature drop was not as pronounced as that of Group A and took place more gradually over the three days of treatment.

The gradual drop in temperature experienced by Group B in spite of these receiving Placebo can be explained by the therapeutic effect of the antibacterial agent which, by reducing the bacterial load, also reduces the pyretic reaction triggered by the organism as a response to the infection.

Over the 10 days at the end of the treatment, clinical symptoms of BRD did not reappear in any of the animals from either treatment group in which the treatment had been assessed as successful by the researcher.

## 3. CONCLUSIONS

From the results obtained in this study, it can be concluded that oral administration of the EV solution in the drinking water, at a dose of 1 ml /10 kg of live weight (equivalent to 3 mg/ kg of its active ingredient, ketoprofen) on three consecutive days, administered complementarily to antibacterial treatment for Bovine Respiratory Disease (BRD), rapidly reduces the clinical symptoms and the percentage of animals cured at the end of the treatment was significantly higher than those obtained when only the antibacterial agent was administered.

## EXAMPLE 3

**STUDY OF THE CLINICAL EFFICACY OF AN AQUEOUS SOLUTION OF KETOPROFEN, ADMINISTERED ORALLY IN ARTIFICIAL MILK FOR LACTATING ANIMALS AS A COMPLEMENT TO ANTIBACTERIAL TREATMENT FOR BOVINE RESPIRATORY DISEASE IN LACTATING CALVES**

This clinical study was carried out to assess the clinical efficacy of the EV solution, a 3% aqueous solution of ketoprofen (Example 1), after its oral administration in artificial milk for suckling animals (milk substitute), as coadjuvant treatment to antibacterial treatment for Bovine Respiratory Disease (BRD) in suckling calf herds.

For this purpose, a controlled multicentric study was used [animals included in the study were distributed into two groups, one received treatment based on Antibiotics + EV solution while the other received a treatment based on Antibiotic + Placebo (Excipient of the EV solution) acting, therefore, as a negative control], ramdomized and blind, in compliance with the principles of Good Clinical Practise.

## 1. MATERIAL AND METHODS

### 1.1 ANIMALS

A total of 188 suckling calves of different breeds and sex were included in the study. According to the selection criteria, at the time of inclusion in the study all the calves presented a clinical picture compatible with moderate or severe BRD.

### 1.2 EXPERIMENTAL DESIGN

The experimental design for this trial was the same as that described in Example 2, section 1.2.

### 1.3 TREATMENTS

#### 1.3.1 Products' characteristics

Both the antibiotic, NUFLOR® (Florfenicol) and the products studied (EV solution and Placebo) were the same as those used in the study described in Example 2, the characteristics of which are described in section 1.3.1 of Example 2. The EV solution and the Placebo were prepared with an identical appearance to ensure the blind character of the study.

#### 1.3.2 Dose, therapeutic regime and route of administration

##### 1.3.2.1 Antibiotic

According to the established protocol the antibiotic preparation was administered to all the animals (groups A and B) according to the dose recommended by the manufacturer by intramuscular injection into the neck muscle. The treatment consisted of 2 doses administered with an interval of 48 h (days D1 and D3).

##### 1.3.2.2 Products studied

In addition to antibiotics, animals in Group A also received a mean dose of 1 ml EV solution /10 kg live weight (equivalent to 3 mg ketoprofen / kg / day) and the animals from Group B a mean dose of 1 ml of Placebo / 10 kg / day. Both products were administered orally mixed in the milk substitute, daily for three consecutive days (D1, D2 and D3).

### 1.4 STATISTICAL PROCEDURES

The same statistical procedures were followed as those described in Example 2, described in section 1.4.

## 2. RESULTS AND DISCUSSION

In spite of the fact that the mean basal value of the clinical index calculated from the previously described parameters was almost identical in both groups, during the control carried out on the second treatment day this value was significantly lower in Group A (treated with EV solution) than in group B (treated with Placebo), remaining this way until the end of treatment (Table 5).

**Table 5**

| Mean values $\pm$ standard deviations of the clinical index for each group during treatment | | | |
|---|---|---|---|
| | GROUP A (EV Solution) | GROUP B (Placebo) | Statistical comparison |
| D1 | $1.77 \pm 0.27$ | $1.78 \pm 0.31$ | NS |
| D2 | $1.47 \pm 0.20$ | $1.60 \pm 0.22$ | $p<0.001$ |
| D3 | $1.07 \pm 0.10$ | $1.13 \pm 0.15$ | $p<0.001$ |
| D4 | $1.01 \pm 0.03$ | $1.03 \pm 0.04$ | $p<0.05$ |
| NS = No statistically significant differences between the two groups ($p>0.05$) | | | |

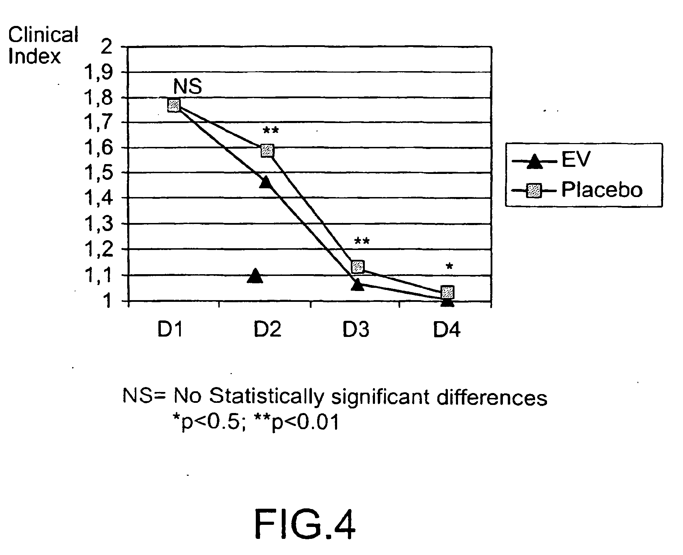

On the other hand, while group A on the third treatment day (D3) reached a mean clinical index lower than 1.1 (equivalent to a low or imperceptible disease status) in Group B this index was still higher than this value by day D4 (Table 5, Figure 4).

These results indicate that the symptoms of the disease disappear earlier in animals from Group A than in animals from Group B. Nevertheless, at the end of the treatment (day D4) almost all animals in both the groups had been cured (Figure 4).

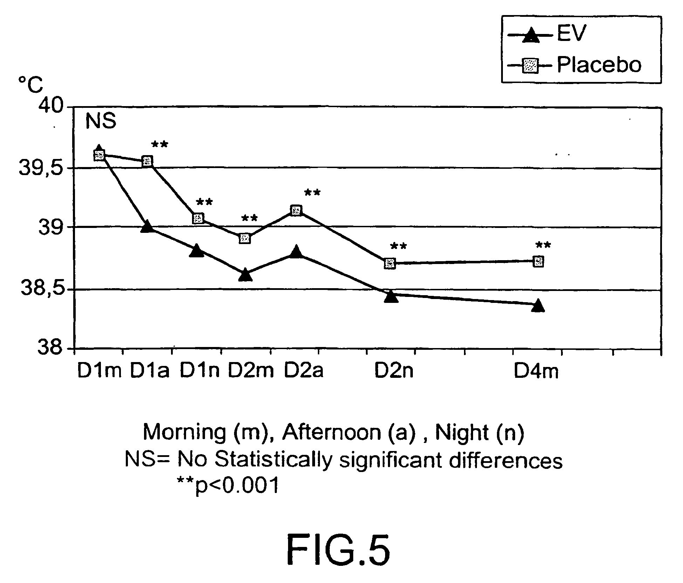

As can be observed in Table 6 and in Figure 5, in spite of the fact that the mean basal temperature was very similar in both groups, on the second day of treatment this was significantly lower in Group A than in Group B and remained this way until the end of the treatment.

**Table 6**

| Mean values $\pm$ standard deviations of the rectal temperatures in each group during treatment | | | |
|---|---|---|---|
| | **GROUP A (EV solution)** | **GROUP B (Placebo)** | **Statistical comparison** |
| **D1-morning** | 39.64 $\pm$ 0.83 °C | 39.60 $\pm$ 0.81 °C | NS |
| **D1-afternoon** | 39.01 $\pm$ 0.55 °C | 39.550 $\pm$ 0.61 °C | P<0.001 |
| **D1-night** | 38.81 $\pm$ 0.47 °C | 39.08 $\pm$ 0.54 °C | P<0.001 |
| **D2-morning** | 38,61 $\pm$ 0,45 °C | 38,91 $\pm$ 0,59 °C | P<0,001 |
| **D2-afternoon** | 38,79 $\pm$ 0,42 °C | 39,15 $\pm$ 0,56 °C | P<0,001 |
| **D3 -morning** | 38.45 $\pm$ 0,50 °C | 38,70 $\pm$ 0,64 °C | P<0,001 |
| **D4 -morning** | 38,38 $\pm$ 0,47 °C | 38,75 $\pm$ 0,52 °C | P<0,001 |
| NS = No statistically significant differences between the two groups (p>0.05) | | | |

As can be observed in Figure 5, there was a considerable drop in temperature in Group A mainly on the first day of treatment.

The gradual temperature drop in group B in spite of these having received Placebo can be explained by the therapeutic effect of the antibacterial agent which reduces the bacterial load that, in turn, reduces the pyretic reaction triggered as a response to infection in the organism.

From Table 7, it can be observed that there are no significant differences in the mean weight presented by both groups of animals on day D4. However, on day D10 animals from Group A presented a significantly higher mean weight than animals from Group B, indicating a greater improvement in clinical status in animals treated with the EV solution. On the other hand, the mean value of the weight increase (calculated from the weight difference between Day 10 and Day 1) in Group A (8.55 $\pm$ 1.18 kg), was also significantly higher (p<0.001) than that of group B (7.50 $\pm$ 1.40 kg).

**Table 7**

| Mean values $\pm$ standard deviation of animal weight on days D1, D4 and D10. | | | |
|---|---|---|---|
| | **GROUP A (EV solution)** | **GROUP B (Placebo)** | **Statistical comparison** |
| **D1** | 77.95 $\pm$ 15.91 kg | 76.00 $\pm$ 18.32 kg | NS |
| **D4** | 80.62 $\pm$ 15.94 kg | 78.52 $\pm$ 18.42 kg | NS |
| **D10** | 86.50 $\pm$ 15.90 kg | 83.50 $\pm$ 18.6 kg | p<0.05 |
| NS = No statistically significant differences between the two groups (p>0.05) | | | |

In the 10 days after the end of the treatment, clinical symptoms of BRD did not reappear in any of the animals from either group in which the treatment had been considered as successful by the researcher.

### 3. CONCLUSIONS

The results obtained in this trial show that the oral administration of EV solution mixed in the milk substitute, at a dose of 1 ml / 10 kg live weight (equivalent to 3 mg/ kg of active ingredient, ketoprofen) for three consecutive days, administered complementarily to antibacterial treatment for BRD in suckling calves significantly helps to alleviate symptoms and to accelerate the animal's recovery.

It can, therefore, be concluded that the EV solution presents a good clinical efficacy as a coadjuvant treatment to antibacterial treatment of Bovine Respiratory Disease in suckling calves.

## EXAMPLE 4

## STUDY OF THE CLINICAL EFFICACY OF AN AQUEOUS SOLUTION OF KETOPROFEN ADMINISTERED ORALLY IN THE DRINKING WATER COMPLEMENTARY TO ANTIBACTERIAL TREATMENT OF PORCINE RESPIRATORY DISEASE COMPLEX

A clinical study was conducted with the aim of evaluating the clinical efficacy of the EV solution of ketoprofen at 3% (Example 1), after its oral administration in the drinking water as a coadjuvant to the antibiotic treatment of Porcine Respiratory Disease Complex (PRDC) in pig herds during fattening.

### 1. MATERIAL AND METHODS

#### 1.1 ANIMALS

Before a pig stockyard was included in the study, the existence of an outbreak of PRDC was confirmed. This diagnosis was made by autopsy of animals found dead in the yard taking samples of lung, lymph nodes and thymus. If no animals had died by the start of the study animals with the worst clinical status were sacrificed. After selecting a pig stockyard, animals that fulfilled the following criteria were selected:

- Pigs in the fattening phase diagnosed with an outbreak of PRDC presenting a rectal temperature over 39.5 °C; and
- animals who had not received antibacterial or antiinflammatory drugs in the seven days before the selection.

At the start of the study, groups of marked animals were distributed into two treatment groups: Group A and Group B, such that each group had a similar number of animals.

During the study, all the animals received a standard pig feed for the fattening phase and drinking water in automatic drinking apparatus connected by a dosifier to special water tanks for the medicated formula.

### 1.2 TREATMENTS

#### 1.2.1 Characteristics of the products

##### 1.2.1.1 Antibiotics

**ALSIR® 5%**

| | |
|---|---|
| Pharmaceutical form: | Injectable solution |
| Composition per ml.: | Enrofloxacin 50 mg |
| | Excipient q.s. |
| Manufacturers: | ESTEVE VETERINARIA, Lab. Dr ESTEVE, S.A. |

##### 1.2.1.2 Products evaluated (A and B)

**Placebo (Product A)**

| | |
|---|---|
| Pharmaceutical form: | Oral solution |
| Composition 100 ml: | Excipient q.s. (Example 1) |
| Manufacturers: | Laboratorios Dr. ESTEVE, S.A. |

**EV solution (Product B)**

| | |
|---|---|
| Pharmaceutical form: | Oral solution |

(continued)

**EV solution (Product B)**

| | |
|---|---|
| Composition 100 ml: | Ketoprofen 3 g (Example 1) |
| | Excipient q.s. |
| Manufacturers: | Laboratorios Dr. ESTEVE, S.A. |

### 1.2.2 Dose, therapeutic regime and route of administration

#### 1.2.2.1 Antibiotics

The antibiotic product was administered by intramuscular route in the dose and therapeutic regime recommended by the manufacturer. Duration of the treatment was 3 days (Days 1, 2 and 3).

#### 1.2.2.2 Products evaluated (A and B)

Both products were administered orally in the drinking water with the following dose and administration regime: 1 ml of Product A or B / 10 kg of live weight (equivalent to 3 mg ketoprofen / kg live weight / day in the case of the treated group). Duration of the treatment was 3 days (Days 1, 2 and 3).

### 1.3 EXPERIMENTAL DESIGN

After selecting a pig stockyard, all the marked animals received an antibiotic treatment of enrofloxacin by intramuscular route. The animals from the yards assigned to one of the treatment groups also received oral treatment with the EV solution added to their drinking water, and those assigned to the other group were administered Placebo (solution excipient) by the same route.

The total study period was 4 days. Treatments were administered on the first three days (D1, D2 and D3). Clinical monitoring of animals was done from the start (D1) until the end (D4) of treatment. Over the study period, four clinical controls were carried out in the morning of each day indicated:

**Controls D1, D2 and D3.** during treatment with antibiotic + Product A or B (control D1 was carried out before the start of the treatment and can be considered as a basal control).

**Control Day 4:** at the end of the treatment.

Several individual and group parameters were measured in each control.

### 1.3.1 Individual parameters

**Clinical Index:** This parameter was measured in controls D1, D2, D3 and D4. This Index was calculated by a score scale based on one described previously [Raynaud (1986), Deleforge (1992), cited *supra*]. This parameter was used to measure the main variable (section 3.3.1).

**Temperature:** This parameter (°C) was measured during controls D1, D2, D3 and D4.

**Weight:** This parameter (kg) was measured in controls D1 and D4.

### 1.3.2 Collective parameters

**General status of the stockyard:** This parameter was measured daily in Controls D1 to D4, on an ordinal scale according to the following scores and evaluating the group of animals together.

**Intake of feed:** This parameter corresponds to the total amount of feed consumed by the animals in each group.

**Mortality:** This parameter corresponds to the number of deaths estimated for each group over the study.

### 1.3.3 Measurement of study variables

#### 1.3.3.1 Main variable

The main study variable can be defined as the "Reduction in clinical index " (RCI).

**1.3.3.2 Secondary variables**

**Rectal temperature**: This variable coincides with the value of the Temperature parameter measured in every control in which it has been recorded.

**Weight increase (WI):** This variable was estimated by applying the following formula:

$$WI: \text{Mean value }_{(marked\ animals)}\ (Weight\ D4 - Weight\ D1)$$

**1.4 STATISTICAL PROCEDURES**

The main study variable "Clinical Index" was analysed by the Student's T test considering a significance level $\alpha = 0.05$ and a power of 80%.

The secondary variables "Temperature" and "Weight" were measured by ANOVA for repeated measures taking a significance level $\alpha = 0.05$ and a power of 80%.

The other parameters measured during the study were analysed by descriptive statistical techniques.

**2. RESULTS AND DISCUSSION**

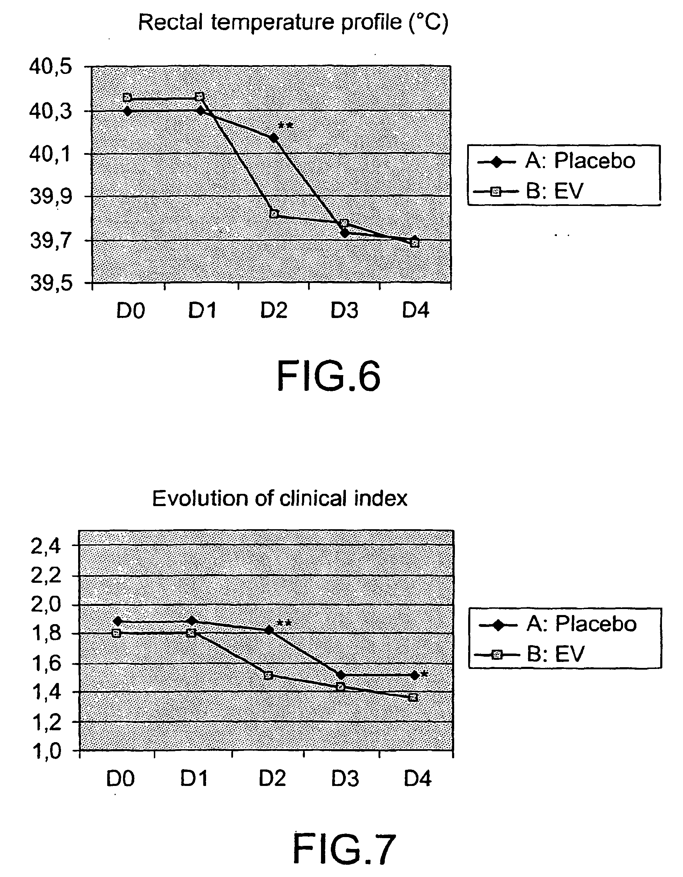

The results corresponding to the rectal temperature profile are shown in Table 8 and Figure 6.

**Table 8**

| Rectal temperature profile | | | | | |
|---|---|---|---|---|---|
| **Rectal temperature (°C)** | | **Basal** | **Day 1** | **Day 2** | **Day 3** |
| A: Placebo | Means | 40.3 | 40.2 | 39.7 | 39.7 |
| | S.D. | 0.4 | 0.4 | 0.3 | 0.3 |
| B: EV solution | Means | 40.4 | 39.8 | 39.8 | 39.7 |
| | S.D. | 0.5 | 0.3 | 0.3 | 0.3 |
| | t-Test | N.S. | P<0.001 | N.S. | N.S. |
| NS = No statistically significant differences between the two groups (p>0.05) | | | | | |

As can be observed in Table 8 and in Figure 6, in spite of the mean basal value of temperature being very similar in both groups, on the second day of treatment this value was significantly lower in Group B (EV solution) than in group A (Placebo). Similarly, from Figure 6, the drop in temperature in group B was pronounced and mainly occurred on the first day of treatment. In contrast, in group A this decline, in addition to it not being as pronounced as for Group B, occurred gradually over the treatment period.

The results obtained for evolution of the clinical index are compiled in Table 9 and Figure 7.

**Table 9**

| Evolution of Clinical Index | | | | | |
|---|---|---|---|---|---|
| **Clinical Index** | | **Basal** | **Day 1** | **Day 2** | **Day 3** |
| A: Placebo | Means | 1.9 | 1.8 | 1.5 | 1.5 |
| | S.D. | 0.4 | 0.4 | 0.4 | 0.3 |
| B: EV solution | Means | 1.8 | 1.5 | 1.4 | 1.4 |
| | S.D. | 0.2 | 0.3 | 0.3 | 0.3 |

(continued)

| Evolution of Clinical Index | | | | | |
|---|---|---|---|---|---|
| Clinical Index | | Basal | Day 1 | Day 2 | Day 3 |
| | t-Test | N.S. | P<0.001 | N.S. | P=0.032 |
| NS = No statistically significant differences between the groups (p>0.05) | | | | | |

From Table 9 and Figure 7, the percentage of animals treated successfully was significantly higher in the group treated with the EV solution than in the group treated with Placebo.

## 3. CONCLUSIONS

According to these results, administration of EV solution to the drinking water as a coadjuvant treatment to the antibacterial treatment of PRDC, presents a beneficial therapeutic effect compared to the treatment of this disease with antibiotics alone.

It can be stated, on the basis of the results of this study, that the EV solution has a good clinical efficacy as a coadjuvant treatment to antibacterial treatment of Porcine Respiratory Disease Complex in fattening pigs.

From the results obtained in the present study, it can be deduced that administration of the EV solution orally in the drinking water, at a dose of 1 ml /10 kg live weight (equivalent to 3 mg/ kg of active ingredient, ketoprofen) for three consecutive days, complementarily to antibacterial treatment of Porcine Respiratory Disease Complex (PRDC) in fattening pigs, results in increased speed of recovery from clinical symptoms and this EV solution presents a good clinical efficacy as a coadjuvant of antibacterial treatment of PRDC in fattening pigs.

**Claims**

1.   Use of ketoprofen for preparing a drinkable preparation comprising drinking water for animals or artificial milk for suckling animals supplemented with ketoprofen as an oral simultaneous coadjuvant treatment to antibacterial treatment of a respiratory disease in a herd of animals.

2.   Use according to claim 1 in which the respiratory disease is Porcine Respiratory Disease Complex (PRDC) in pigs and Bovine Respiratory Disease (BRD) in calves.

3.   Use according to claim 2 in which the ketoprofen is administered as a coadjuvant to an antibacterial treatment.

4.   Use according to any of claims 1-3 in which the ketoprofen is previously dissolved in an aqueous solution.

5.   Use according to claim 4 in which the concentration of ketoprofen in this aqueous solution of ketoprofen ranges from 1 to 15 % (w/v).

6.   Use according to any of claims 4 or 5 in which this aqueous solution comprises a co-solvent.

7.   Use according to claim 6, in which this co-solvent is selected from among polyethylene glycol 400 and 1-methyl-2-pyrrolidone.

8.   Use according to any of claims 4-7 in which this aqueous solution includes a basic amino acid capable of forming a salt *in situ* with ketoprofen and increasing the solubility of ketoprofen.

9.   Use according to claim 8, in which this basic amino acid is selected from L-lysine and L-arginine.

10.  Use according to any of claims 4-9 in which this aqueous solution comprises one or more veterinary acceptable additives, selected from humectant agents, pH modifiers, preservatives agents and combinations thereof.

11.  Use according to claim 10, in which this humectant agent is a polysorbate or a poloxamere.

**12.** Use according to claim 10, in which this pH modifier is a compound, acidic or basic, that provides an aqueous solution of ketoprofen with a pH of between 5.5 and 7.0.

**13.** Use according to claim 10, in which this preservative agent is benzylic alcohol or a parabene.

**14.** Use according to any of claims 4-13, in which this aqueous solution of ketoprofen has the following composition:

| Ingredient | |
| --- | --- |
| Ketoprofen | 1-15% (w/v) |
| L-Arginine | 2-30% (w/v) |
| Benzylic alcohol | 0.5-3% |
| Anhydrous citric acid q.s. | pH from 5.5 to 7.0 |
| Purified water q.s. | 100% |

**15.** Use according to claim 14, in which this aqueous solution of ketoprofen has the following composition:

| Ingredient | |
| --- | --- |
| Ketoprofen | 1-5% (w/v) |
| L-Arginine | 2-8% (w/v) |
| Benzylic alcohol | 0.5-2.5% |
| Anhydrous citric acid q.s. | pH 6-7 |
| Purified water q.s. | 100% |

**16.** Use according to claim 4-12 in which this aqueous solution of ketoprofen has the following composition:

| Ingredient | |
| --- | --- |
| Ketoprofen | 1-15% (w/v) |
| L-Arginine | 2-30% (w/v) |
| Anhydrous citric acid q.s. | pH 5.5 - 7.0 |
| Purified water q.s. | 100% |

**17.** An aqueous solution for preparing a drinkable preparation comprising drinking water for animals or artificial milk for suckling animals supplemented with ketoprofen having the following composition:

| Ingredient | |
| --- | --- |
| Ketoprofen | 1-15% (w/v) |
| L-Arginine | 2-30% (w/v) |
| Benzylic alcohol | 0.5-3% |
| Anhydrous citric acid q.s. | pH from 5.5 to 7.0 |
| Purified water q.s. | 100% |

**18.** An aqueous solution according to claim 17 having the following composition:

| Ingredient | |
| --- | --- |
| Ketoprofen | 1-5% (w/v) |
| L-Arginine | 2-8% (w/v) |
| Benzylic alcohol | 0.5-2.5% |

(continued)

| Ingredient | |
| --- | --- |
| Anhydrous citric acid q.s. | pH 6-7 |
| Purified water q.s. | 100% |

**19.** An aqueous solution for preparing a drinkable preparation comprising drinking water for animals or artificial milk for suckling animals supplemented with ketoprofen having the following composition:

| Ingredient | |
| --- | --- |
| Ketoprofen | 1-15% (w/v) |
| L-Arginine | 2-30% (w/v) |
| Anhydrous citric acid q.s. | pH from 5.5 to 7.0 |
| Purified water q.s. | 100% |

**20.** Method for obtaining a drinkable preparation supplemented with ketoprofen comprising the mixture of an aqueous solution of ketoprofen according to any of claims 17 to 19 with drinking water for animals or artificial milk for suckling animals.

**Patentansprüche**

**1.** Verwendung von Ketoprofen zur Herstellung einer trinkbaren Zubereitung, die Trinkwasser für Tiere oder künstliche Milch zum Säugen von Tieren, ergänzt durch Ketoprofen, enthält, für eine orale gleichzeitige Coadjuvans-Behandlung zu einer antibakteriellen Behandlung einer Atemwegserkrankung bei einer Gruppe von Tieren.

**2.** Verwendung nach Anspruch 1, bei der es sich bei der Atemwegserkrankung handelt um den Schweine-Atemwegserkrankungs-Komplex (PRDC) bei Schweinen und um die Rinder-Atemwegserkrankung (BRD) bei Kälbern.

**3.** Verwendung nach Anspruch 2, bei der das Ketoprofen als ein Coadjuvans zu einer antibakteriellen Behandlung verabreicht wird.

**4.** Verwendung nach einem der Ansprüche 1 bis 3, bei der das Ketoprofen vorher in einer wässrigen Lösung aufgelöst wird.

**5.** Verwendung nach Anspruch 4, bei der die Ketoprofen-Konzentration in der wässrigen Ketoprofen-Lösung in dem Bereich von 1 bis 15 Gew./Vol.-% liegt.

**6.** Verwendung nach einem der Ansprüche 4 oder 5, bei der die wässrige Lösung ein Colösungsmittel umfasst.

**7.** Verwendung nach Anspruch 6, bei der dieses Colösungsmittel ausgewählt wird aus der Gruppe Polyethylenglycol 400 und 1-Methyl-2-pyrrolidon.

**8.** Verwendung nach einem der Ansprüche 4 bis 7, bei der diese wässrige Lösung eine basische Aminosäure enthält, die in der Lage ist, mit Ketoprofen in situ ein Salz zu bilden und die Löslichkeit von Ketoprofen zu erhöhen.

**9.** Verwendung nach Anspruch 8, bei der diese basische Aminosäure ausgewählt wird aus der Gruppe L-Lysin und L-Arginin.

**10.** Verwendung nach einem der Ansprüche 4 bis 9, bei der diese wässrige Lösung ein oder mehr veterinärmedizinisch akzeptable Additive, ausgewählt aus Feuchthaltemitteln, pH-Modifizierungsmitteln, Konservierungsmitteln und Kombinationen davon, umfasst.

**11.** Verwendung nach Anspruch 10, bei der das Feuchthaltemittel ein Polysorbat oder ein Poloxamer ist.

**12.** Verwendung nach Anspruch 10, bei der das pH-Modifizierungsmittel eine saure oder basische Verbindung ist, die eine wässrige Lösung von Ketoprofen mit einem pH-Wert zwischen 5,5 und 7,0 ergibt.

**13.** Verwendung nach Anspruch 10, bei der das Konservierungsmittel Benzylalkohol oder ein Paraben ist.

**14.** Verwendung nach einem der Ansprüche 4 bis 13, bei der die wässrige Ketoprofen-Lösung die folgende Zusammensetzung hat:

| Bestandteil | |
| --- | --- |
| Ketoprofen | 1 bis 15 Gew./Vol.-% |
| L-Arginin | 2 bis 30 Gew./Vol.-% |
| Benzylalkohol | 0,5 bis 3 % |
| wasserfreie Citronensäure q.s. | pH-Wert 5,5 bis 7,0 |
| gereinigtes Wasser q.s. | 100 % |

**15.** Verwendung nach Anspruch 14, bei der die wässrige Ketoprofen-Lösung die folgende Zusammensetzung hat:

| Bestandteil | |
| --- | --- |
| Ketoprofen | 1 bis 5 Gew./Vol.-% |
| L-Arginin | 2 bis 8 Gew./Vol.-% |
| Benzylalkohol | 0,5 bis 2,5 % |
| wasserfreie Citronensäure q.s. | pH-Wert 6 bis 7 |
| gereinigtes Wasser q.s. | 100 % |

**16.** Verwendung nach einem der Ansprüche 4 bis 12, bei der die wässrige Ketoprofen-Lösung die folgende Zusammensetzung hat:

| Bestandteil | |
| --- | --- |
| Ketoprofen | 1 bis 15 Gew./Vol.-% |
| L-Arginin | 2 bis 30 Gew./Vol.-% |
| wasserfreie Citronensäure q.s. | pH-Wert 5,5 bis 7,0 |
| gereinigtes Wasser q.s. | 100 % |

**17.** Wässrige Lösung zur Herstellung einer trinkbaren Zubereitung, die Trinkwasser für Tiere oder künstliche Milch zum Säugen von Tieren, ergänzt durch Ketoprofen, enthält und die folgende Zusammensetzung hat:

| Bestandteil | |
| --- | --- |
| Ketoprofen | 1 bis 15 Gew./Vol.-% |
| L-Arginin | 2 bis 30 Gew./Vol.-% |
| Benzylalkohol | 0,5 bis 3 % |
| wasserfreie Citronensäure q.s. | pH-Wert 5,5 bis 7,0 |
| gereinigtes Wasser q.s. | 100 % |

**18.** Wässrige Lösung nach Anspruch 17 mit der folgenden Zusammensetzung:

| Bestandteil | |
| --- | --- |
| Ketoprofen | 1 bis 5 Gew./Vol.-% |

(fortgesetzt)

| Bestandteil | |
| --- | --- |
| L-Arginin | 2 bis 8 Gew./Vol.-% |
| Benzylalkohol | 0,5 bis 2,5 % |
| wasserfreie Citronensäure q.s. | pH-Wert 6 bis 7 |
| gereinigtes Wasser q.s. | 100 % |

**19.** Wässrige Lösung zur Herstellung einer trinkbaren Zubereitung, die Trinkwasser für Tiere oder künstliche Milch zum Säugen von Tieren, ergänzt durch Ketoprofen, enthält und die folgende Zusammensetzung hat:

| Bestandteil | |
| --- | --- |
| Ketoprofen | 1 bis 15 Gew./Vol.-% |
| L-Arginin | 2 bis 30 Gew./Vol.-% |
| wasserfreie Citronensäure q.s. | pH-Wert 5,5 bis 7,0 |
| gereinigtes Wasser q.s. | 100 % |

**20.** Verfahren zur Herstellung einer trinkbaren Zubereitung, ergänzt durch Ketoprofen, die umfasst eine Mischung einer wässrigen Ketoprofen-Lösung nach einem der Ansprüche 17 bis 19 mit Trinkwasser für Tiere oder künstlicher Milch zum Säugen von Tieren.

**Revendications**

**1.** Utilisation de kétoprofène pour la préparation d'une préparation buvable comprenant de l'eau potable, pour des animaux ou du lait artificiel pour des animaux non sevrés complété des kétoprofène en tant que traitement coadjuvant simultané oral pour un traitement antibactérien d'une maladie respiratoire dans un cheptel d'animaux.

**2.** Utilisation selon la revendication 1, dans laquelle la maladie respiratoire est le Complexe de la Maladie Respiratoire du Porcin (PRDC) chez les cochons et la Maladie Respiratoire Bovine (BRD) chez les veaux.

**3.** Utilisation selon la revendication 2, dans laquelle le kétoprofène est administré en tant que coadjuvant pour un traitement antibactérien.

**4.** Utilisation selon l'une quelconque des revendications 1-3 dans laquelle le kétoprofène est au préalable dissous dans une solution aqueuse.

**5.** Utilisation selon la revendication 4, dans laquelle la concentration en kétoprofène dans cette solution aqueuse de kétoprofène est comprise entre 1 et 15% (p/v).

**6.** Utilisation selon l'une quelconque des revendications 4 ou 5, dans laquelle la solution aqueuse comprend un co-solvant.

**7.** Utilisation selon la revendication 6, dans laquelle ce co-solvant est sélectionné parmi du polyéthylène glycol 400 et de la 1-méthyl-2-pyrrolidone.

**8.** Utilisation selon l'une quelconque des revendications 4-7 dans laquelle cette solution aqueuse contient un acide aminé basique capable de former un sel *in situ* avec du kétoprofène et d'augmenter la solubilité du kétoprofène.

**9.** Utilisation selon la revendication 8, dans laquelle l'acide aminé basique est sélectionné parmi L-lysine et L-arginine.

**10.** Utilisation selon l'une quelconque des revendications 4-9 dans laquelle cette solution aqueuse comprend un ou plusieurs additifs acceptables en médecine vétérinaire, sélectionnés parmi des agents humectants, des agents modificateurs du pH, des agents conservateurs et leurs combinaisons.

**11.** Utilisation selon la revendication 10, dans laquelle cet agent humectant est un polysorbate ou un poloxamère.

**12.** Utilisation selon la revendication 10, dans laquelle ce modificateur du pH est un composé acide ou basique, qui produit une solution aqueuse de kétoprofène ayant un pH compris entre 5,5 et 7,0.

**13.** Utilisation selon la revendication 10, dans laquelle l'agent conservateur est de l'alcool benzylique ou un parabène.

**14.** Utilisation selon l'une quelconque des revendications 4-13, dans laquelle la solution aqueuse de kétoprofène a la composition qui suit:

| Ingrédient | |
|---|---|
| Kétoprofène | 1-15% (p/v) |
| L-Arginine | 2-30% (p/v) |
| Alcool benzylique | 0,5-3% |
| Acide citrique anhydre s.q. | pH de 5,5 à 7,0 |
| Eau purifiée s.q. | 100% |

**15.** Utilisation selon la revendication 14, dans laquelle cette solution aqueuse de kétoprofène a la composition qui suit:

| Ingrédient | |
|---|---|
| Kétoprofène | 1-15% (p/v) |
| L-Arginine | 2-8% (p/v) |
| Alcool benzylique | 0,5-2,5% |
| Acide citrique anhydre s.q. | pH 6-7 |
| Eau purifiée s.q. | 100% |

**16.** Utilisation selon la revendication 4-12 dans laquelle la solution aqueuse de kétoprofène a la composition qui suit:

| Ingrédient | |
|---|---|
| Kétoprofène | 1-15% (p/v) |
| L-Arginine | 2-30% (p/v) |
| Acide citrique anhydre s.q. | pH de 5,5 à 7,0 |
| Eau purifiée s.q. | 100% |

**17.** Solution aqueuse pour la préparation d'une préparation à boire comprenant de l'eau potable pour des animaux ou du lait artificiel pour des animaux non sevrés complété de kétoprofène ayant la composition qui suit:

| Ingrédient | |
|---|---|
| Kétoprofène | 1-15% (p/v) |
| L-Arginine | 2-30% (p/v) |
| Alcool benzylique | 0,5-3% |
| Acide citrique anhydre s.q. | pH de 5,5 à 7,0 |
| Eau purifiée s.q. | 100% |

**18.** Solution aqueuse selon la revendication 17 ayant la composition qui suit:

| Ingrédient | |
| --- | --- |
| Kétoprofène | 1-5% (p/v) |
| L-Arginine | 2-8% (p/v) |
| Alcool benzylique | 0,5-2,5% |
| Acide citrique anhydre s.q. | pH 6-7 |
| Eau purifiée s.q. | 100% |

**19.** Solution aqueuse pour préparer une préparation à boire comprenant de l'eau potable pour des animaux ou du lait artificiel pour des animaux non sevrés complété de kétoprofène ayant la composition qui suit:

| Ingrédient | |
| --- | --- |
| Kétoprofène | 1-15% (p/v) |
| L-Arginine | 2-30% (p/v) |
| Acide citrique anhydre s.q. | pH de 5,5 à 7,0 |
| Eau purifiée s.q. | 100% |

**20.** Méthode pour obtenir une préparation à boire complétée de kétoprofène comprenant le mélange d'une solution aqueuse de kétoprofène selon l'une quelconque des revendications 17 à 19 avec de l'eau potable pour des animaux ou du lait artificiel pour des animaux non sevrés.

TREATMENT

| DAY 1 | D2 | D3 | D4 | D5 | D6 | ........................ | D10 |

FIG.1

EP 1 462 101 B1

NS= No Statistically significant differences
*p<0.05; **p<0.001

FIG.2

°C

NS= No Statistically significant differences
*p<0.05; **p<0.001

FIG.3

NS= No Statistically significant differences
*p<0.5; **p<0.01

# FIG.4

FIG.5

Morning (m), Afternoon (a) , Night (n)
NS= No Statistically significant differences
**p<0.001

Rectal temperature profile (°C)

FIG.6

Evolution of clinical index

FIG.7